(19) Europäisches Patentamt
European Patent Office
Office européen des brevets

(11) Publication number: **0 457 529 B1**

(12) **EUROPEAN PATENT SPECIFICATION**

(45) Date of publication of patent specification: **09.08.95**

(51) Int. Cl.6: **A61B 5/0408**, H01B 1/20

(21) Application number: **91304274.3**

(22) Date of filing: **13.05.91**

(54) **An electroconductive and astringent film and film-forming composition for application to skin.**

(30) Priority: **17.05.90 US 525331**

(43) Date of publication of application:
**21.11.91 Bulletin 91/47**

(45) Publication of the grant of the patent:
**09.08.95 Bulletin 95/32**

(84) Designated Contracting States:
**AT BE CH DE DK ES FR GB IT LI NL SE**

(56) References cited:
**US-A- 4 215 696**

**Gantrez ES Monoester Resins 1977, GAF
Corp. Wayne, New Jersey, USA page 1,
Functional Properties, Physical and Chemi-
cal Properties; page 7, Typical Formulations**

**DATABASE WPIL, abstract-no. 892-93 340E
concerning to JP-A-57-55 132, DERWENT
PUBLICATIONS LTD., London UK**

**DATABASE WPIL, abstract no. 88-53 321 con-
cerning to JP-A-63-9 425, DERWENT PUB-
LICATIONS LTD., London, UK**

(73) Proprietor: **SMITH & NEPHEW UNITED, INC.
11775 Starkey Road,
P.O. Box 1970
Largo,
Florida 34649-1970 (US)**

(72) Inventor: **Scheps, Milton Harold
11775 Starkey Road,
P.O. Box 1970
Largo,
Florida 34649-1970 (US)**
Inventor: **Saute, Robert
11775 Starkey Road,
P.O. Box 1970
Largo,
Florida 34649-1970 (US)**

(74) Representative: **Gilholm, Stephen Philip
Corporate Patents Department
Smith & Nephew Group Research Centre
York Science Park
Heslington
York YO1 5DF (GB)**

**Description**

The present invention relates to films which are rendered electroconductive and astringent by the addition of an ionic astringent compound and to compositions for application to the human skin to provide a film which has electroconductive and astringent properties.

When using an electromechanical apparatus to monitor or stimulate a part of the anatomy, it is essential that the contact between the tissue and the electrode connected to the monitoring or stimulation apparatus has a low resistance and provides a high degree of conductivity. To achieve these desirable objectives the electrode contact area should be free of skin oil and other contaminants. United States Patent No. 3710782, for example, describes a method of preparing the skin for electrode attachment using an aerosol containing an alcohol, an electrolyte and propellant. This composition is applied to the skin, left for a few seconds and then removed to leave no residue on the skin. However, it has been observed that when an electrode with a dry pad is used, there is a risk of the skin beneath the electrode pad becoming wet so causing failure of the adhesive adhering the electrode to the skin even when the skin had been deoiled and cleaned.

In US Patent No. 4215696 there is disclosed a biomedical electrode with pressurised skin contact which *inter alia* comprises a dome-like chanter bridged, at its open bottom by a flexible, microporous, enformable membrane which is adapted to be adhesively attached to the skin. Within the housing of the dome is contained an electrolyte. Electrically conductive contact between the electrolyte and the skin is established through the porous membrane. The electrolyte is typically a thixotropic gel and may include an electrolyte salt such as $Zn(OAc)_2$.

In Japanese Publication No. 80-JO-130272 there is described a transparent gel composition for use in reducing contact resistance between an electrode and the skin which gel is comprised of polymer mono- and di-sodium phosphates, propylene glycol and water. The gel may be readily removed by wiping off the skin.

It has been suggested that a polymer film might be used as an intermediate layer between the electrode and the skin beneath the film may still become hydrated and secondly the film may not be sufficiently conductive. The present invention seeks to overcome these problems by the provision of an ionic astringent which prevents hydration of the skin beneath the applied electrode and by the ionic nature of the compound, confers electroconductive properties to the film.

In accordance with the present invention there is provided a composition, suitable for application to the skin comprising a mixture of film-forming amount of a film-forming polymer, an electroconductive amount of an ionic astringent compound and a carrier therefor said ionic astringent compound excluding zinc acetate and zinc lactate, being substantially homogeneously dispersed through the mixture.

By substantially homogeneously dispersed, it is meant that the ionic astringent compound is soluble in or is in the form of very fine particles in the film so that the average density of the compound is substantially the same throughout the film.

The compositions of the invention are capable of providing a water-proof, highly conductive film on the skin surface to which may be applied electronic monitoring and transcutaneous electronic nerve stimulation (TENS) apparatus.

The films are observed not to reduce the electronic signal received from an electrode by a monitoring apparatus when compared to the signal received from an electrode placed on clean skin. The electrodes adhere well to the film over a long period of time. The film is observed to prevent redness and hydration of the skin beneath the electrode.

Suitably the ionic astringent compound is present in an amount of at least from about 0.25% by weight of the total composition. Generally we have found that the amount of astringent need not be greater than 3.0 by weight. An apt range therefor may be in the range of from 0.25 to 3.0 by weight of the total composition. Preferably, it is present in an amount of from about 0.40% to 2.5% by weight, particularly from about 0.40% to about 1.5%.

Suitable ionic astringent compounds are organic compounds containing zinc or aluminium ions. Zinc salts are particularly useful.

The preferred astringent ionic compound is zinc phenolsulphonate because this compound has been found to be particularly effective.

The astringent ionic compound should be compatible with the film forming polymer preferably to the extent that the film formed on the skin is transparent.

The film-forming polymers employed in the present invention may be used in an amount of at least about 3% to weight of the total composition. Generally, the amount of film-forming polymer employed the present invention need not exceed about 20% by weight of the total composition. An apt range is from 3 to 20% by weight of the composition preferably from 5 to 16%, more preferably from about 7 to about 14%.

The film forming polymer employed in the present invention may be any of those which will form films on skin and which when formed are conformable to movement of the skin.

Aptly the polymer is a polymer which contains carboxyl groups as it has been found that such polymers are compatible with ionic astringents and allow for easier homogeneous dispersion to take place.

A preferred polymer is a copolymer of methylvinylether and maleic acid since this provides a film having excellent electroconductive and astringent properties in the presence of an ionic astringent such as zinc phenolsulfonate.

The film-forming copolymer of methylvinylether and maleic acid is usually present in the form of its alkyl ester preferably an alkyl monoester of 1 to 6 carbon atoms. The butyl ester has been found to be particularly suitable. Gantrez ES-435 of GAF Corporation is a suitable film-forming copolymer and is the butyl monoester of poly(methylvinylether and maleic acid).

A useful film-forming polymer is the monobutylester of a copolymer of methylvinyl ether and maleic acid.

The composition may additionally contain a plasticising amount of a plasticiser for the film-forming polymer and may be present in an amount of at least 0.1% by weight of the total composition. When a plasticiser is employed, the amount is aptly not greater than about 3% by weight of the total composition. The amount of plasticiser may therefor range from about 0.1% to about 3% by weight of the total composition, preferably about 0.3% to about 1.5%.

The plasticiser may aptly be a citric acid ester for example acetyl tributyl citrate, sold under the trade name CITROFLEX A-4.

A suitable carrier for the astringent and film-forming component of the components of the present invention comprises an alcohol. An apt alcohol is isopropyl alcohol. Preferably the alcohol carriers are employed as mixtures thereof in water. A suitable carrier is a water isopropyl alcohol mixture, for example 70% aqueous Isopropyl alcohol. The alcohol may be present in the amounts of at least about 70% by weight of the total composition. Aptly the alcohol carrier maybe employed in an amount upto 90% by weight of the total composition. Suitably isopropyl alcohol may be present in an amount of from 70 to 90% by weight of the total composition.

The compositions of the present invention may additionally contain an antimicrobially effective amount of an antimicrobial agent.

Suitably the antimicrobial agent is present in an effective amount, aptly at least 0.1% by weight of the total composition. Antimicrobial agents may be employed in amounts upto 2.0% by weight.

Suitable antimicrobial agents are 2,4,4'trichloro-2'-hydroxydiphenyl ether (available under the trade name TRICLOSAN) zinc and aluminium phenolsulfonates, benzethonium chloride and related quaternary compounds, butyl paraben, chloroacetimide and o-phenyl phenol.

The film-forming polymer and ionic astringent compound are aptly both soluble in the carrier. This facilitates the homogeneous dispersion of the astringent compound. The astringent compound should be distributed homogeneously to ensure effective manifestation of the films electroconductive and astringent properties. The film-forming material and ionic astringent compound should be compatible since disassociation of the two components, for example by precipitation, may also lead to loss of electroconductivity and astringent properties for the film.

A useful composition according to the present invention comprises from about 3% to about 20% by weight of the butylmonoester of poly(methylvinylether and maleic acid), from about 0.25% to about 3% by weight zinc phenolsulfonate, from about 70% to about 90% by weight isopropyl alcohol, from about 0.1% to about 3% by weight acetyl tributyl citrate and from about 0.1% to about 2% by weight of an antimicrobial agent.

Preferably the electroconductive astringent film formed from the compositions of the present invention are transparent.

The compositions of the present invention are prepared by thoroughly mixing the ingredients according to procedures per se known in the art. Tanks, homogeniser and other manufacturing equipment that come into contact with the ingredients of the composition should generally be washed with a hot detergent solution, rinsed, sanitized with an antiseptic such as a chlorine solution and again rinsed prior to use.

The composition of the present invention may be produced in different form such as a brush-on liquid or an aerosol spray or impregnated onto an absorbent material such as a swab or paper.

A brush-on liquid may be prepared by slowly adding the antimicrobial agent if one is desired to be present such as triclosan to an alcohol such as an aqueous alcohol solution for example isopropyl alcohol, preferably 70% aqueous and thoroughly mixing the two until dissolution of the antimicrobial agent in the alcohol is complete. Then the plasticiser, for example, a citric acid ester, the astringent, for example zinc phenolsulfonate and the polymer for example the copolymer methylvinylether and maleic acid are then

EP 0 457 529 B1

added and mixed until dissolution is complete and a homogeneous solution is formed. If the antimicrobial agent is desired, the alcohol is added to plasticiser and the copolymer added as above described.

An aerosol spray is prepared by adding to the above composition a conventional propellant such as a hydrocarbon, for example propane or butane, or a fluorinated hydrocarbon.

Commonly, equal amounts of the composition and the propellant are used.

An impregnated swab is forced by soaking the sway material in the liquid composition described above and packaging the impregnated swab in a water-proof pouch.

The following non-limitative examples more particularly illustrate the present invention.

Example 1

The following ingredient are combined in the percentages by weight indicated below:

| | |
|---|---|
| **Acetyl tributyl citrate** | **0.70%** |
| **Triclosan** | **0.25%** |
| **Gantrez ES-435** | **14.00%** |
| **Isopropyl alcohol** | **70.00%** |
| **Zinc phenolsulfonate** | **0.50%** |
| **Water** | **14.55%** |

Isopropyl alcohol and water are added to a presantised, stainless steel vessel equipped with turbine agitation. Triclosan in slowly added and mixed well until complete dissolution. The acetyl tributyl citrate, Gantrez ES-435 and zinc phenolsulfonate are added and mixed thoroughly for complete dissolution and to obtain a homogeneous solution.

Example 2

The following ingredients are combined in the percentages by weight indicated below:

| | |
|---|---|
| Medicated skin preparation of Example 1 except for isopropyl alcohol and water | 24.0% |
| Isopropyl alcohol | 26.0% |
| Propellant A-45([1]) | 50.0% |

([1]) mixture of propane and butane under a pressure of 46 pounds (3.3 bar) at 70°F (21°C).

The medicated skin preparation and the isopropyl alcohol are mixed with a high speed mixer for 15 minutes. The mixture is filled into cans with an aerosol filling machine. The propellant is added to each can with the filling machine. Each can contains 4 3/8 oz (124.0gm) of product of which 2 3/16 oz (62.0gm) is the medicated skin preparation and 2 3/16 oz (62.0gm) is the propellant.

When the above described compositions are applied in an continuous coating manner to human skin they dry to form a bacterial or microbial barrier which is highly electroconductive and which prevents the build up of sweat or moisture beneath the film. The films can be taped over as desired and can have electronic monitoring or transcutaneous electronic nerve stimulation apparatus applied to them. The presence of the film does not affect the conduction of electronic signals between the skin and the apparatus. The presence of the zinc phenolsulfonate also prevents the build up of sweat beneath the film. Removal of the tape or apparatus results in a bifurcation of the composition into two layers. One layer adheres to the tape or apparatus and is removed with same. The other layer adheres to the human skin on which it may be left to degrade or it may be washed off. In place of triclosan, zinc and aluminium phenolsulfonates, benzethonium chloride and related quaternary compounds, butyl paraben, chloroacetimide and o-phenyl phenol may be used.

4

**Claims**

1. A composition suitable for application to the skin to provide thereon a film with electroconductive and astringent properties, which composition comprises a mixture of a film-forming amount of a film-forming polymer, an electroconductive amount of an ionic astringent compound and a carrier therefor said ionic astringent compound excluding zinc acetate and zinc lactate, being substantially homogeneously dispersed throughout the mixture.

2. A composition according to claim 1 wherein the ionic astringent compound is present in an amount of from about 0.25% to about 3% by weight of the total composition.

3. A composition according to claim 1 or claim 2 wherein the ionic astringent compound is zinc phenolsulfonate.

4. A composition according to any one of claims 1 to 3 wherein the amount of film-forming polymer is from 3% to 20% by weight of the total composition.

5. A composition according to any one of claims 1to 4 wherein the film-forming polymer is the monobutylester of a copolymer of methylvinyl ether and maleic acid.

6. A composition according to any one of claims 1 to 5 which additionally contains a plasticising amount of plasticiser for the film-forming polymer.

7. A composition according to claim 6 wherein the amount of plasticiser is from 0.1% to 3% by weight of the total composition.

8. A composition according to any one of claims 1 to 7 wherein the carrier is an alcohol.

9. A composition according to claim 8 wherein the alcohol is isopropyl alcohol.

10. A composition according to claim 9 wherein the isopropyl alcohol is a water isopropyl alcohol mixture.

11. A composition according to claim 10 wherein the isopropyl alcohol is present in the amount of from about 70% to about 90% by weight of the total composition.

12. A composition according to any one of claims 1 to 10 which additionally contains an antimicrobially effective amount of an antimicrobial agent.

13. A composition according to claim 12 wherein the antimicrobial agent is 2,4,4′tri-trichloro-2′-hydroxydiphenyl ether, zinc phenolsulfonate, aluminium phenolsulphonate, benzethonium chloride, butyl paraben, chloroacetimide or o-phenyl phenol.

14. A composition according to claim 12 or claim 13 wherein the antimicrobial agent is present in an amount of from 0.05% to 2%.

15. A composition according to claim 1 which comprises from 3% to 20% by weight of the butylmonoester of poly(methylvinylether and maleic acid), from 0.25% to 3% by weight zinc phenolsulfonate, from 70% to 90% by weight isopropyl alcohol, from 0.1% to 3% by weight acetyl tributyl citrate and from 0.1% to 2% by weight of an antimicrobial agent.

16. A composition according to any one of claims 1 to 15 which is transparent.

17. An electroconductive astringent film formed from the composition claimed in any one of claims 1 to 16.

**Patentansprüche**

1. Zur Anwendung auf der Haut geeignete Zusammensetzung, um darauf einen Film mit elektrisch leitenden und adstringierenden Eigenschaften bereitzustellen, wobei die Zusammensetzung ein Gemisch aus einer filmbildenden Menge eines filmbildenden Polymers, einer elektrisch leitfähigen Menge einer adstringierenden ionischen Verbindung und einem Träger dafür umfaßt, wobei die adstringierende ionische Verbindung ausgenommen Zinkacetat und Zinklactat in dem ganzen Gemisch im wesentlichen homogen dispergiert ist.

2. Zusammensetzung gemäß Anspruch 1, wobei die adstringierende ionische Verbindung in einer Menge von etwa 0,25 Gew.-% bis etwa 3 Gew.-% der gesamten Zusammensetzung vorliegt.

3. Zusammensetzung gemäß Anspruch 1 oder Anspruch 2, wobei die adstringierende ionische Verbindung Zinkphenolsulfonat ist.

4. Zusammensetzung gemäß einem der Ansprüche 1 bis 3, wobei die Menge an filmbildendem Polymer von 3 Gew.-% bis 20 Gew.-% der gesamten Zusammensetzung beträgt.

5. Zusammensetzung gemäß einem der Ansprüche 1 bis 4, wobei das filmbildende Polymer der Monobutylester eines Copolymers aus Methylvinylether und Maleinsäure ist.

6. Zusammensetzung gemäß einem der Ansprüche 1 bis 5, welche zusätzlich eine weichmachende Menge eines Weichmachers für das filmbildende Polymer enthält.

7. Zusammensetzung gemäß Anspruch 6, wobei die Menge an Weichmacher von 0,1 Gew.-% bis 3 Gew.-% der gesamten Zusammensetzung beträgt.

8. Zusammensetzung gemäß einem der Ansprüche 1 bis 7, wobei der Träger ein Alkohol ist.

9. Zusammensetzung gemäß Anspruch 8, wobei der Alkohol Isopropylalkohol ist.

10. Zusammensetzung gemäß Anspruch 9, wobei der Isopropylalkohol ein Wasser-Isopropylalkohol-Gemisch ist.

11. Zusammensetzung gemäß Anspruch 10, wobei der Isopropylalkohol in einer Menge von etwa 70 Gew.-% bis etwa 90 Gew.-% der gesamten Zusammensetzung vorhanden ist.

12. Zusammensetzung gemäß einem der Ansprüche 1 bis 10, welche zusätzlich eine antimikrobiell wirksame Menge eines antimikrobiellen Mittels enthält.

13. Zusammensetzung gemäß Anspruch 12, wobei das antimikrobielle Mittel 2,4,4'-Trichlor-2'-hydroxydiphenylether, Zinkphenolsulfonat, Aluminiumphenolsulfonat, Benzethoniumchlorid, Butylparaben, Chloracetimid oder o-Phenylphenol ist.

14. Zusammensetzung gemäß Anspruch 12 oder Anspruch 13, wobei das antimikrobielle Mittel in einer Menge von 0,05% bis 2% vorliegt.

15. Zusammensetzung gemäß Anspruch 1, welche von 3 Gew.-% bis 20 Gew.-% des Butylmonoesters von Poly(methylvinylether und Maleinsäure), von 0,25 Gew.-% bis 3 Gew.-% Zinkphenolsulfonat, von 70 Gew.-% bis 90 Gew.-% Isopropylalkohol, von 0,1 Gew.-% bis 3 Gew.-% Acetyltributylcitrat und von 0,1 Gew.-% bis 2 Gew.-% eines antimikrobiellen Mittels enthält.

16. Zusammensetzung gemäß einem der Ansprüche 1 bis 15, welche durchsichtig ist.

17. Elektrisch leitender, adstringierender Film, welcher aus einer in einem der Ansprüche 1 bis 16 beanspruchten Zusammensetzung gebildet ist.

**Revendications**

1. Composition appropriée à l'application sur la peau, afin d'y produire un film doté de propriétés électroconductrices et astringentes, laquelle composition comprend un mélange d'une quantité génératrice de film, d'un polymère générateur de film, d'une quantité électroconductrice d'un composé ionique astringent et d'un véhicule approprié, ledit composé ionique astringent (à l'exclusion de l'acétate de zinc ou du lactate de zinc), étant dispersé de façon pratiquement homogène au sein du mélange.

2. Composition selon la revendication 1, dans laquelle le composé ionique astringent est présent en quantité comprise entre environ 0,25 % et environ 3 % en poids, par rapport à la composition totale.

3. Composition selon la revendication 1, ou la revendication 2, dans laquelle le composé ionique astringent est le phénolsulfonate de zinc.

4. Composition selon l'une quelconque des revendications 1 à 3, dans laquelle la quantité de polymère générateur de film est comprise entre 3 % et 20 % en poids, par rapport à la composition totale.

5. Composition selon l'une quelconque des revendications 1 à 4, dans laquelle le polymère générateur de film est un copolymère de méthylvinyl éther et d'acide maléique.

6. Composition selon l'une quelconque des revendications 1 à 5, qui en plus, contient une quantité plastifiante d'un plastifiant pour le polymère générateur de film.

7. Composition selon la revendication 6, dans laquelle la quantité de plastifiant est comprise entre 0,1 % et 3 % en poids, par rapport à la composition totale.

8. Composition selon l'une quelconque des revendications 1 à 7, dans laquelle le véhicule est un alcool.

9. Composition selon la revendication 8, dans laquelle l'alcool est l'alcool isopropylique.

10. Composition selon la revendication 9, dans laquelle l'alcool isopropylique est un mélange d'eau et d'alcool isopropylique.

11. Composition selon la revendication 10, dans laquelle l'alcool isopropylique est présent en quantité comprise entre environ 70 % et environ 90 % en poids, par rapport à la composition totale.

12. Composition selon l'une quelconque des revendications 1 à 10, qui contient en plus, une quantité efficace du point de vue antimicrobien, d'un agent antimicrobien.

13. Composition selon la revendication 12, dans laquelle l'agent antimicrorobien est le 2,4,4'-tri-trichloro-2'-hydroxydiphényl éther, le phénolsulfonate de zinc, le phénolsulfonate d'aluminium, le chlorure de benzéthonium, le butylparaben, le chloracétamide, ou l'o-phénylphénol.

14. Composition selon la revendication 12, ou la revendication 13, dans laquelle l'agent antimicrobien est présent en quantité comprise entre 0,05 % et 2 %.

15. Composition selon la revendication 1, qui comprend de 3 % à 20 % en poids, de butylmonoester de poly(méthylvinyléther et acide maléique), de 0,25 % à 3 % en poids, de phénolsulfonate de zinc, de 70 % à 90 % en poids, d'alcool isopropylique, de 0,1 % à 3 % en poids, d'acétyle tributyle citrate, et de 0,1 % à 2 % en poids, d'un agent antimicrobien.

16. Composition selon l'une quelconque des revendications 1 à 15, qui est transparente.

17. Film astringent électroconducteur formé à partir d'une composition selon l'une quelconque des revendications 1 à 16.